# NOUVEAU FASCICULE DE BREVET EUROPEEN

(11) **EP 1 095 925 B2**
(45) Date de publication et mention de la décision concernant l'opposition: **02.05.2012**
(45) Mention de la délivrance du brevet: 20.08.2003
(21) Numéro de dépôt: 00402952.6
(22) Date de dépôt: 25.10.2000
(51) Int. Cl.: C07C 31/26, C07C 29/88, C07C 29/141, A61K 8/60, C07C 29/76

(54) **Procédé de préparation d'un sirop de polyols non cristallisable**
Verfahren zur Herstellung eines nichtkristallisierbaren Polyolsirups
Process for the preparation of a non crystallizing polyol syrop

(30) Priorité: 28.10.1999 FR 9913492
(43) Date de publication de la demande: 02.05.2001
(73) Titulaire: Roquette Frères, 62136 Lestrem (FR)
(72) Inventeur: Salome, Jean-Paul, 59232 Vieux-Berquin (FR); Ferez, Patrick, 62136 Lestrem (FR); Lefevre, Philippe, 59400 Merville (FR)
(74) Mandataire: Boulinguiez, Didier

(56) Documents cités:
- EP-A- 0 711 743
- CH-A5- 592 141
- US-A- 4 329 183
- US-A- 5 127 956
- US-A- 5 254 174
- PATENT ABSTRACTS OF JAPAN vol. 1995, no. 09, 31 octobre 1995 (1995-10-31) & JP 07 145090 A (TOWA CHEM IND CO LTD), 6 juin 1995 (1995-06-06)
- K.J.Steinbach et al., Mitt.vgl.Nahrung 5, 66 (1961)
- J.N.Bemiller et R.K. Mann, Carbohydrate Research 2 (1966), pages 70-79
- Handbuch Süssungsmittel-Eigenschaften und Anwendung - G.-W.von Rymon Lipinski, H.Schiweck (1991), 383
- Glucose Syrups:Science and Technology (1984), p.117-121, Chapter 3: Production of hydrogenated Glucose Syrups and some Aspects of their Use F.Verwaerde and P.J.Sicard

## Description

L'invention a pour objet un procédé de préparation d'un sirop de polyols non cristallisable, stable à la chaleur et aux alcalis.

Elle a plus précisément trait à un procédé de préparation d'un sirop de polyols susceptible d'être utilisé dans la fabrication de lessives, de détergents, dans la formulation de sirops pharmaceutiques, de pâtes dentifrices, c'est à dire dans toute application qui nécessite une résistance en milieu alcalin et/ou à chaud face à la formation de colorations indésirables ou de composés inadaptés du point de vue de leur goût.

On entend par polyols les produits obtenus par hydrogénation catalytique de sucres réducteurs simples, de sucres réducteurs complexes tels que les disaccharides, oligo-saccharides et polysaccharides, ainsi que leurs mélanges, que l'on désignera par la suite par le terme " sirop de sucres ".

De façon générale, les sucres réducteurs simples que l'on destine à l'hydrogénation catalytique selon l'invention sont le glucose, le xylose, le fructose et le mannose. Les polyols obtenus sont alors le sorbitol, le xylitol et le mannitol.

Les disaccharides sont le plus souvent le maltose, l'isomaltulose, le maltulose, l'isomaltose et le lactose, qui conduisent par hydrogénation catalytique, au maltitol, à l'isomalt, à l'isomaltitol et au lactitol.

Par composition non cristallisable, on entend dans la présente invention les mélanges de polyols qui forment des sirops incristallisables à 20°C et à une matière sèche de 70% lorsqu'ils sont stockés dans un récipient hermétique à l'air durant un mois. A titre indicatif, la définition d'un sirop de sorbitol non cristallisable au sens de l'invention est conforme à la pharmacopée européenne, 1997,paragraphe 0437.

Les sirops de sorbitol sont largement employés dans les domaines agro-alimentaires, pharmaceutiques et chimiques. Dans la formulation de pâtes dentifrices et en particulier dans la fabrication de pâtes dentifrices au bicarbonate de sodium, l'utilisation de sirops de sorbitol en tant qu'humectant n'est possible que si ce dernier est stable en présence du bicarbonate de sodium, et n'engendre pas de coloration brune au stockage. En effet, cette coloration apparaît par réaction du bicarbonate sur les sucres réducteurs : glucose, maltose, oligo- et polysaccharides. L'intensité de la coloration augmente avec le nombre d'unités du polysaccharide, c'est ainsi qu'une molécule de maltose engendrera plus de coloration qu'une molécule de dextrose. La coloration est également fortement accélérée par la température à laquelle est exposé le sirop. On a en effet remarqué qu'une même intensité de coloration est obtenue lors d'un stockage de 10 jours à 45°C et lors d'un stockage de 15 mois à 20°C. On préfère en outre que ces sirops soient non cristallisables, afin de faciliter leur manipulation et leur transport quelles que soient les conditions climatiques et d'assurer la stabilité dans le temps des produits finis préparés à partir de tels sirops.

Les sirops de maltitol représentent également une classe importante, et sont utilisés principalement pour préparer des produits alimentaires et pharmaceutiques non cariogènes.

Les sirops de xylitol, bien que plus coûteux, connaissent un développement important en raison de leur pouvoir sucrant élevé, de leurs propriétés dentaires et de leurs excellentes caractéristiques humectantes.

Il est connu que la coloration d'un sirop de polyols en présence d'alcalis est liée à la présence de sucres libres, réducteurs : glucose, maltose, oligo- et polysaccharides.

L'amélioration de la stabilité de tels sirops passe donc par l'élimination de ces sucres libres.

Plusieurs voies ont déjà été envisagées à l'échelle du laboratoire.

Le brevet japonais JP 51.86406 offre des perspectives d'amélioration de la pureté de sirops de sorbitol cristallisables en mettant en jeu une réduction de glucose cristallisé sous alcalinité entretenue durant toute la réaction de réduction, dans le but d'obtenir du sorbitol de grande pureté pauvre en sucres non réduits.

Toutefois, cette technique reste dépourvue d'intérêt pratique à l'échelle industrielle car elle nécessite la mise en place d'un dispositif d'alimentation, de contrôle et de régulation onéreux, et l'addition constante de tampon et de solution alcaline au cours de la réaction pénalise l'étape de purification ultérieure. Ce procédé est en outre relativement polluant compte tenu des quantités importantes de réactifs utilisés. De plus, rien n'est dit sur la stabilité des sirops de sorbitol résultant dudit procédé.

Le brevet japonais JP 41.12212 fait mention d'un procédé de préparation de sorbitol de pureté maximale résistant à la chaleur et aux alcalis, qui consiste en une réduction par addition d'hydrogène à haute pression, en prévoyant soit de régler la solution à pH 8 à 10 juste avant que la réaction de réduction ne s'achève, soit de porter à une température de 60 à 90°C la solution de sorbitol réduite et préalablement réglée à pH 8 à 10 au moyen d'un alcali, puis après décomposition des sucres directement réducteurs résiduels, en une séparation par filtration avec ou sans neutralisation par un acide et avec ou sans décoloration sur charbon, puis en une purification sur résine échangeuse d'ions.

Ce procédé, qui s'applique à une solution de sorbitol cristallisable, donne lieu à une importante formation d'impuretés du fait que les sucres réducteurs subissent un temps de traitement relativement long de l'ordre de cinq heures, et n'aboutit donc pas à une stabilité jugée suffisante.

Les brevets japonais JP 63.79844 et JP 7.145090 décrivent un procédé de préparation de polyols stables à la chaleur et aux alcalis, qui consiste à traiter pendant une à deux heures à chaud et en milieu alcalin une solution aqueuse de polyols purifiée une première fois par traitement au noir puis sur résine échangeuse d'ions, puis à purifier une nouvelle fois la solution obtenue à 50°C, par passage sur résine échangeuse d'ions. Ce procédé s'adresse tout particulièrement à dès sirops de sorbitol cristallisables, obtenus à partir de sirops de glucose de haute pureté. Or de tels sirops cristallisables ne sont pas adaptés à une utilisation notamment dans des pâtes dentifrices en raison des nombreux inconvénients qu'ils présentent. D'autre part, ce procédé s'avère particulièrement complexe du fait des multiples opérations dont il est constitué, et donc difficilement applicable à l'échelle industrielle.

Le brevet EP 0 711 743, dont la demanderesse est titulaire décrit des compositions de polyols présentant une grande stabilité chimique en milieu alcalin et une très faible réactivité.

Ces compositions, particulièrement adaptées à une utilisation en milieu basique nécessitant une absence de coloration, sont obtenues par hydrogénation catalytique de sucres réducteurs simples ou complexes, puis par stabilisation et purification du sirop stabilisé.

L'étape de stabilisation consiste à soumettre les sirops de sorbitol obtenus par hydrogénation à une oxydation, une caramélisation ou une fermentation, de manière à amener les sirops à une densité optique inférieure ou égale à 0.100 dans un test S.

La demanderesse avait en effet démontré qu'une stabilité satisfaisante ne pouvait être atteinte qu'à de faibles valeurs dans ce test, celui-ci reflétant l'aptitude des compositions à la coloration en milieu alcalin.

Cherchant à améliorer encore les performances d'un tel procédé, et dans un souci de limiter les rejets minéraux et organiques pour préserver l'environnement, la demanderesse a alors mis au point, après de longs travaux de recherche, un procédé permettant à la fois d'obtenir des sirops de polyols non cristallisables suffisamment stables en milieu alcalin, et d'offrir aisément en limitant les opérations de purification un rendement élevé et une pollution moindre, ce que ne permettaient pas d'obtenir les techniques classiques connues de l'art antérieur.

C'est ainsi que la demanderesse a trouvé qu'il convenait, afin d'obtenir des sirops de polyols non cristallisables stables à la chaleur et aux alcalis, de soumettre un sirop de sucres ayant subi une étape d'hydrogénation et de caramélisation, à une étape de purification sur au moins une résine cationique forte à une température inférieure à 30°C ladite température étant choisie en fonction du taux de sucres réducteurs recherché dans la composition finale.

L'invention a donc pour objet un nouveau procédé de préparation d'un sirop de polyols non cristallisable stable à la chaleur et en milieu alcalin, mettant en oeuvre une étape d'hydrogénation d'un sirop de sucres et une étape de caramélisation du sirop de sucres hydrogéné, caractérisé par le fait que le sirop de sucres hydrogéné et caramélisé subit une purification sur résines échangeuses d'ions, ladite purification comprenant au moins un passage sur résine cationique forte à une température inférieure à 30°C ladite température étant choisie en fonction du taux de sucres réducteurs recherché dans le sirop de polyols non cristallisable et la caramélisation est conduite dans le réacteur d'hydrogénation, sous hydrogène et sans séparation du catalyseur, par introduction d'un agent alcalin en fin de réaction d'hydrogénation, sans ajout de tampon, lorsque la teneur en sucres réducteurs résiduels est inférieure à 0,2 %.

La demanderesse a en effet démontré après de nombreux travaux l'importance de la température de travail sur le maintien de la qualité du produit lors de la purification sur résine cationique forte. En effet, la qualité de la composition après purification et en particulier son taux de sucres réducteurs final est inversement proportionnel à la température de passage sur résine cationique forte.

La purification sur résine cationique forte selon l'invention est donc conduite à une température inférieure à 30°C, choisie en fonction du taux de sucres réducteurs désiré dans la composition finale après purification. En effet, la Demanderesse a trouvé que l'on pouvait régler la température de passage sur résine en fonction d'une part du taux-de sucres réducteurs atteint après caramélisation et d'autre part de la teneur en sucrés réducteurs finale voulue pour le sirop de polyols purifié. Cette teneur recherchée varie en fonction des applications visées pour le sirop. Pour la préparation de pâtes dentifrices, outre la nature de l'agent alcalin présent dans la pâte, il faut notamment tenir compte de la nature des colorants utilisés pour déterminer la limite acceptable de résistance à la coloration pour le sirop de polyols. En effet, une légère coloration jaune sera plus facilement admise pour des pâtes colorées en bleu. Les arômes utilisés sont également un facteur à prendre en compte pour déterminer la limite du taux de sucres réducteurs acceptable. Ainsi, pour certaines pâtes aux bicarbonates, la limite acceptable est de 350 parties par million de sucres réducteurs (exprimée en équivalents dextrose et désignée ci-après par ppm), et pour d'autres comme celles contenant des pyrophosphates, des taux allant jusqu'à 500 ppm peuvent convenir.
Ceci est également applicable pour d'autres applications pharmaceutiques, cosmétiques ou alimentaires.

Dans des conditions optimales de conduite d'une hydrogénation puis d'une caramélisation d'un sirop de polyols non cristallisable, on obtient des valeurs minimales de sucres réducteurs de l'ordre de 50 à 100 ppm. Dans ces conditions, la Demanderesse a trouvé que la température maximale à laquelle on puisse purifier un tel sirop sur résine cationique forte est de 30°C. En deçà de 30°C, compte tenu de l'accroissement du taux de sucres réducteurs dans les resines, on peut donc régler la température de passage sur la résine en fonction du taux de sucres réducteurs désiré dans la composition finale et du taux de sucres réducteurs initialement présents après caramélisation, comme il sera développé ultérieurement. C'est ainsi que le passage sur résine cationique forte peut être réglé à une température inférieure à 30°C et plus préférentiellement encore comprise entre 20 et inférieure à 30°C si l'on recherche un très faible taux de sucres réducteurs dans la composition de polyols selon l'invention.

La purification en elle-même est effectuée selon les pratiques courantes, c'est à dire qu'on opère en premier lieu à un passage sur résine cationique forte puis sur résine anionique forte, puis sur lit mixte qui est un mélange partie pour partie de ces deux résines. Il est également possible de modifier l'ordre de combinaison de ces résines.

La résine cationique forte a pour but d'éliminer les cations tels que notamment le sodium apporté par la soude utilisée lors de la caramélisation, et le nickel soluble apporté par le catalyseur d'hydrogénation.

La résine anionique forte a pour but d'éliminer les anions organiques tels que notamment le gluconate, qui est un produit de dégradation issu de l'étape de caramélisation.

L'utilisation en dernière étape d'un lit mixte permet d'optimiser la purification en palliant les éventuelles fuites d'ions qui auraient pu se produire lors des étapes précédentes.

On préfère utiliser comme échangeur de cations une résine cationique forte portant un groupement fonctionnel de type sulfonique SO3H utilisée sous forme acide fort, telle que par exemple la résine IR 200 C commercialisée par ROHM et HAAS. En ce qui concerne la résine anionique, on préfère utiliser une résine anionique forte telle que la résine IRA 910, commercialisée par le même fabricant. Le lit mixte sera constitué d'un mélange des deux résines.

Selon un mode de réalisation avantageux du procédé conforme à l'invention, on effectue la purification sur résines à un débit correspondant à 1,5 fois le volume de la colonne de résine traversée par le sirop par heure, ceci afin d'éviter des temps de séjour trop longs dans la résine qui risquent de favoriser la dégradation de la qualité du sirop de polyols purifié.

La caramélisation est conduite dans le réacteur d'hydrogénation, sous hydrogène et sans séparation du catalyseur, par introduction d'un agent alcalin en fin de réaction d'hydrogénation, à un moment ou le pH est susceptible d'être stable après ajout de cet agent alcalin sans pour cela avoir recours à un tampon.

La demanderesse a en effet trouvé après de nombreux travaux que l'étape d'hydrogénation pouvait être avantageusement combinée à une caramélisation dans le même réacteur, sans ajout de tampon, de manière à obtenir de façon économique et peu polluante après purification, une composition de polyols stable en milieu alcalin et à chaud à partir d'un sirop de sucres.

On entend par caramélisation au sens de la présente invention une dégradation alcaline des sucres réducteurs de l'hydrogénat, conduisant à la formation d'énols correspondants. Parmi les agents alcalins convenant bien à la caramélisation, on peut citer les bases fortes ou faibles. Selon un mode de réalisation préféré, l'agent alcalin utilisé pour la caramélisation est la soude.

Le sirop de sucres soumis au procédé selon l'invention peut consister notamment en sirops de glucose, de fructose, en sirops de glucose riches en maltose, ou encore en sirops de xylose.

De façon avantageuse, le sirop de sucres est constitué par 60 à 95% de dextrose, 0.1 à 20 % de maltose, le complément à 100 étant constitué de poly et oligo-saccharides, ces pourcentages étant exprimés en poids par rapport au poids sec des saccharides contenus dans ledit sirop.

L'hydrogénation catalytique est réalisée de façon connue en soi, dans un réacteur à double enveloppe, sur des catalyseurs au nickel de Raney, tout autre catalyseur d'hydrogénation des sucres pouvant convenir.

De préférence, elle est effectuée à une pression d'hydrogène comprise entre 30 et 100 bars, à une température comprise entre 120 et 150°C, et encore plus préférentiellement à une température comprise entre 130 et 150°C, ceci afin d'optimiser la vitesse d'hydrogénation tout en limitant les réactions secondaires.

De la soude est introduite dans le réacteur de manière à obtenir un pH compris entre 9 et 11, de préférence compris entre 9,5 et 11, à un moment où celui-ci est suffisamment stable pour ne pas avoir recours à une solution tampon ou pour ne pas devoir ajouter une quantité massive de soude afin de maintenir le pH à cette valeur. Ce stade est généralement atteint au bout de 1 heure 30 d'hydrogénation, dans les conditions conformes à l'invention.

Ce critère d'introduction de la soude résulte de l'étude des cinétiques d'hydrogénation et des conditions de caramélisation. Lorsque le milieu réactionnel est encore riche en sucres réducteurs libres, l'introduction de l'agent alcalin entraîne une instabilité du pH qui chute sensiblement du fait de la conversion de ces sucres réducteurs libres en acides correspondants. Ainsi, au-delà d'une teneur en sucres réducteurs d'environ 0.4%, on observe une formation trop importante d'acides et donc une chute de pH rendant inefficace l'action stabilisatrice de la caramélisation et impliquant un ajout trop important de soude. On introduit donc l'agent alcalin lorsque la teneur en sucres réducteurs résiduels est inférieure à 0.2%, et plus préférentiellement encore inférieure ou égale à 0.1%.

Lorsque le pH dans le réacteur est inférieur à 9, la caramélisation est insuffisante. Lorsque celui-ci est supérieur à 11, la caramélisation est satisfaisante mais la charge ionique de l'hydrogénat devient trop importante ce qui entraîne un rejet conséquent de chlorures lors de la régénération des résines échangeuses de cations. A un pH compris entre 9,5 et 11, la demanderesse a constaté que l'excès de soude dans le milieu réactionnel était suffisant pour assurer une caramélisation complète des sucres.

Le procédé conforme à l'invention permet d'obtenir des sirops de polyols particulièrement adaptés à une utilisation dans la préparation de produits de pH basique tels que, notamment, les pâtes dentifrices à base de bicarbonate de sodium ou de la famille des phosphates de sodium, les compositions antiacides, les mousses à raser, les crèmes épilatoires, ou pour la fabrication de produits à haute température, tout en atteignant une rentabilité inégalée à ce jour, et un niveau de rejets organiques et minéraux minimal.

Selon un mode de réalisation avantageux du procédé conforme à l'invention, le sirop obtenu est un sirop de sorbitol non cristallisable.

Préférentiellement, le sirop de sorbitol non cristallisable obtenu présente une teneur en sorbitol d'au moins 64% en poids, une teneur en maltitol d'au moins 6% en poids, la teneur en oligo- et polysaccharides constituant le complément à 100%, ces pourcentages étant exprimés par rapport à la matière sèche des polyols présents dans la composition.

Le sirop susceptible d'être obtenu selon le procédé conforme à l'invention peut ainsi être avantageusement utilisé dans la préparation de produits à pH basique, contenant des agents alcalins, ou traités ou obtenus à haute température.

L'invention sera illustrée à l'aide des exemples qui suivent.

### Exemple 1

Dans un réacteur à double enveloppe d'une capacité de 20 litres, contenant du nickel de Raney en suspension, on introduit sous agitation un sirop de sucres dont la composition est la suivante :
- dextrose : 75% / sec
- maltose : 8% / sec
- maltotriose : 3.6% / sec
- DP supérieurs : 13.4% / sec
La matière sèche du milieu réactionnel est de 40% en poids, et la teneur en Nickel de Raney est de 5% en poids, exprimés par rapport au poids sec.

L'hydrogénation est conduite pendant 1h30 à une pression de 50 bars et une température de 140°C.

On introduit alors une solution de soude à 3% en poids pendant 15 minutes de manière à amener le pH de l'hydrogénat une valeur de 10.8. On constate que le pH est stable, la teneur en sucres réducteurs étant inférieure à 0.4% en poids.

On poursuit l'hydrogénation pendant 20 minutes.

Après quoi on arrête l'agitation du réacteur, on laisse décanter pendant 15 minutes et on vidange le surnageant vers un décanteur afin de récupérer le catalyseur. Le surnageant du décanteur est ensuite filtré afin d'éliminer les dernières traces de catalyseur.

On soumet le sirop ainsi obtenu, après l'avoir refroidi à 25°C, à une purification sur résine cationique forte, puis sur résine anionique, puis sur lit mixte.

On soumet ensuite le sirop obtenu à un test de stabilité aux agents alcalins. Ce test, intitulé test S est décrit dans le brevet EP 711 743 dont la demanderesse est titulaire. La stabilité des compositions de polyols est d'autant plus importante que la valeur obtenue dans ce test S est basse (densité optique inférieure à 0,1).

A 5ml de sirop, on ajoute 500 mg d'hydrogénocarbonate de sodium de qualité ultrapure, et 250 mg d'une solution aqueuse à 20% d'ammoniac.

On mélange l'ensemble et on le chauffe pendant 2 heures au bain-marie à 100°C sans agitation.

On refroidit la solution à 20°C et on mesure la densité optique de celle-ci à une longueur d'onde de 420 nm, à l'aide d'un spectrophotomètre tel que celui commercialisé par PERKIN-ELMER sous la marque Lambda 5 UV/VIS Spectrophotometer.

De la même manière, on réalise une gamme d'étalonnage en remplaçant les 5ml de sirop par 3ml d'eau pure et 2ml de solutions de glucose pur anhydre, de concentration 100, 200, 300, 400, 500, 600 et 1000 parties par million.

Ces solutions de glucose ont pour absorbance respectivement : 0.04, 0.08, 0.120, 0.160, 0.205, 0.250, 0.413.

On obtient pour le sirop obtenu conformément à la présente invention une densité optique relativement basse, de 0,04.

Ceci équivaut à un titre en glucose de 100 parties par million sur sec, ce qui est une valeur indicative d'une très grande stabilité aux alcalis.

Le procédé conforme à l'invention permet donc d'obtenir de façon plus économique et moins polluante que les techniques de l'art antérieur une composition de polyols non cristallisable, très stable en milieu alcalin et/ou à chaud.

### Exemple 2 : Influence de la température sur la purification au moyen de résines échangeuses d'ions.

On reprend le sirop obtenu selon l'exemple 1 avant purification, que l'on divise en sept fractions.

Ces fractions sont purifiées sur résine cationique forte, respectivement à 20, 30, 35, 40, 45, 50, 52 et 60°C (fractions identifiées de A à H), puis sur résine anionique et enfin sur lit mixte.

On effectue sur chaque fraction un test S après purification et on calcule pour chaque point la différence avec le test initial (delta test S).

Les résultats sont donnés en parties par million équivalent dextrose.

| TEMPERATURE DE PURIFICATION | FRACTION | Taux de sucres réducteurs après purification exprimés en ppm équivalent dextrose | Accroissement du taux de sucres réducteurs (en ppm équ. dextrose) |
|---|---|---|---|
| 20°C | A | 100 | 0 |
| Comparatif 30°C | B | 200 | 100 |
| " 35°C | C | 260 | 160 |
| " 40°C | D | 350 | 250 |
| " 45°C | E | 450 | 350 |
| " 50°C | F | 560 | 460 |
| " 52°C | G | 600 | 500 |
| " 60°C | H | 950 | 850 |

Ces résultats démontrent clairement l'influence de la température de travail lors de la purification sur résine cationique forte. On montre ainsi qu'il est possible d'adapter la température de la composition lors de sa purification, selon les exigences en matière de sucres réducteurs que l'application finale visée nécessite, ce qui procure au procédé selon l'invention une souplesse jusqu'alors inexistante.

### Exemple 3 : formulation d'une pâte dentifrice au bicarbonate de sodium.

Une pâte dentifrice au bicarbonate de sodium est réalisée avec les produits A et F de l'exemple 2 (purifiés sur résines à 20°C et à 50°C) selon la formule suivante :

| | PATE A | PATE B |
|---|---|---|
| | (% en poids) | |
| Sirop A | 45.00 | - |
| Sirop F | | 45.00 |
| Bicarbonate de sodium | 10.00 | 10.00 |
| Silice abrasive Tixosil 73 | 9.00 | 9.00 |
| Silice abrasive Tixosil 43 | 10.00 | 10.00 |
| Lauryl sulfate de sodium | 5.66 | 5.66 |
| (solution aqueuse à 30%) | | |
| Monofluorophosphate de sodium | 0.80 | 0.80 |
| Carboxymethylcellulose sodique | 0.70 | 0.70 |
| Dioxyde de titane | 0.70 | 0.70 |
| Arôme menthe | 1.00 | 1.00 |
| Eau purifiée | qs 100.00 | id |
| Saccharinate de sodium | 0.2 | 0.2 |

Les pâtes dentifrices A et B telles qu'obtenues présentent respectivement un pH de 8,4, et de 8,7 en solution à 10%.

Après stockage de six mois à température ambiante, la couleur de la pâte A n'a pas évolué en raison de la pureté satisfaisante du produit A.

La pâte B présente par contre une couleur jaunâtre inacceptable après le même temps de stockage.

## Revendications

1. Procédé de préparation d'un sirop de polyols non cristallisable, stable à la chaleur et en milieu alcalin, mettant en oeuvre une étape d'hydrogénation d'un sirop de sucres et une étape de caramélisation du sirop de sucres hydrogéné, **caractérisé par le fait que** le sirop de sucres hydrogéné et caramélisé subit une purification sur résines échangeuses d'ions, ladite purification comprenant au moins un passage sur résine cationique forte à une température inférieure à 30 °C, ladite température étant choisie en fonction du taux de sucres réducteurs recherché dans le sirop de polyols non cristallisable, et **par le fait que** la caramélisation est conduite dans le réacteur d'hydrogénation, sous hydrogène et sans séparation du catalyseur, par introduction d'un agent alcalin en fin de réaction d'hydrogénation, sans ajout de tampon, lorsque la teneur en sucres réducteurs résiduels est inférieure à 0,2 %.

2. Procédé selon la revendication 1, **caractérisé par le fait que** le traitement de caramélisation est conduit dans le réacteur d'hydrogénation, sous hydrogène et sans séparation du catalyseur, par introduction de soude en fin de réaction d'hydrogénation.

3. Procédé selon l'une quelconque des revendications 1 et 2, **caractérisé par le fait que** l'étape de caramélisation consiste à amener le pH du sirop hydrogéné à une valeur comprise entre 9 et 11, de préférence comprise entre 9,5 et 11, lorsque le pH est susceptible d'être stable.

4. Procédé selon l'une quelconque des revendications 1 à 3, **caractérisé par le fait que** l'étape de purification est conduite sur résine cationique forte à une température inférieure à 30 °C, puis sur résine anionique et enfin sur lit mixte.

5. Procédé selon l'une quelconque des revendications 1 à 4, **caractérisé par le fait que** la température de passage sur résine cationique forte est comprise entre 20 °C et inférieure à 30 °C.

6. Procédé selon l'une quelconque des revendications 1 à, 5, **caractérisé par le fait que** le sirop de polyols est un sirop de sorbitol non cristallisable.

7. Procédé selon la revendication 6, **caractérisé par le fait que** le sirop de sorbitol non cristallisable a une teneur en sorbitol d'au moins 64 % en poids, une teneur en maltitol d'au moins 6 % en poids, la teneur en oligo- et polysaccharides constituant le complément à 100 %, ces pourcentages étant exprimés par rapport à la matière sèche des polyols présentes dans la composition.

## Claims

1. Process for preparing a noncrystallizable polyol syrup stable to heat and to alkaline medium, using a step of hydrogenation of a sugar syrup and a step of caramelization of the hydrogenated sugar syrup, **characterized in that** the hydrogenated and caramelized sugar syrup is subjected to purification on ionexchange resins, the said purification comprising at least one passage over a strong cationic resin at a temperature of less than 30°C, the said temperature being chosen according to the level of reducing sugars desired in the noncrystallizable polyol syrup, and **in that** the caramelization is performed in the hydrogenation reactor, under hydrogen and without separation of the catalyst, by introducing an alkaline agent at the end of the hydrogenation reaction, without adding a buffer, when the reducing sugar content is lower than 0.2%.

2. Process according to Claim 1 **characterized in that** the caramelization treatment is performed in the hydrogenation reactor, under hydrogen and without separation of the catalyst, by introducing sodium hydroxide at the end of the hydrogenation reaction.

3. Process according to either of Claims 1 and 2, **characterized in that** the caramelization step consists in bringing the pH of the hydrogenated syrup to a value of between 9 and 11, preferably of between 9.5 and 11 when this pH is likely to be stable.

4. Process according to any one of Claims 1 to 3, **characterized in that** the purification step is performed on a strong cationic resin at a temperature of less than 30°C, and then on an anionic resin and finally on a mixed bed.

5. Process according to any one of Claims 1 to 4, **characterized in that** the temperature for passing over a strong cationic resin is between 20 and less than 30°C.

6. Process according to any one of Claims 1 to 5, **characterized in that** the polyol syrup is a noncrystallizable sorbitol syrup.

7. Process according to Claim 6, **characterized in that** the noncrystallizable sorbitol syrup has a sorbitol content of at least 64% by weight, the oligo- and polysaccharide content constituting the balance for 100%, these percentages being expressed relative to the dry matter content of the polyols present in the composition.

## Patentansprüche

1. Verfahren zur Herstellung eines nicht kristallisierbaren, gegenüber Hitze und im alkalischen Milieu beständigen Polyolsirups, umfassend einen Hydrierschritt eines Zuckersirups und einen Karamellisierschritt des Sirups von hydrierten Zuckern, **dadurch gekennzeichnet, dass** der Sirup von hydrierten und karamelisierten Zuckern einer Reinigung über lonenaustauschharzen unterzogen wird, die Reinigung mindestens eine Passage über ein starkes Kationenharz bei einer Temperatur unter 30°C umfasst, wobei die Temperatur in Abhängigkeit vom gewünschten Gehalt an reduzierenden Zuckern in dem nicht kristallisierbaren Polyolsirup ausgewählt wird, und **dadurch** dass die Karamellisierung im Hydrierreaktor durchgeführt wird, unter Wasserstoff und ohne Katalysatorabtrennung, durch Einbringen eines alkalischen Mittels am Schluss der Hydrierreaktion, ohne Zusatz eines Puffers, wenn der Gehalt an reduzierenden Zuckern geringer als 0,2% ist.

2. Verfahren nach Anspruch 1, **dadurch gekennzeichnet, dass** die Karamellisierbehandlung im Hydrierreaktor durchgeführt wird, unter Wasserstoff und ohne Katalysatorabtrennung, durch Einbringen von Soda am Schluss der Hydrierreaktion.

3. Verfahren nach einem der Ansprüche 1 und 2, **dadurch gekennzeichnet, dass** der Karamellisierschritt darin besteht, den pH-Wert des hydrierten Sirups auf einen Wert zwischen 9 und 11, bevorzugt zwischen 9,5 und 11 zu bringen, wenn dieser pH-Wert für Stabilisierung empfänglich ist.

4. Verfahren nach einem der Ansprûche 1 bis 3, **dadurch gekennzeichnet, dass** der Reinigungsschritt über starkes Kationenharz bei einer Temperatur unter 30°C, danach über Anionenharz und schließlich über Mischbett durchgeführt wird.

5. Verfahren nach einem der Ansprüche 1 bis 4, **dadurch gekennzeichnet, dass** die Temperatur der Passage über starkes Kationenharz zwischen 20 und unter 30°C beträgt.

6. Verfahren nach einem der Ansprüche 1 bis 5, **dadurch gekennzeichnet, dass** das Polyolsirup ein nicht kristallisierbares Sorbitolsirup ist.

7. Verfahren nach Anspruch 6, **dadurch gekennzeichnet, dass** das nicht kristallisierbare Sorbitolsirup einen Sorbitolgehalt von mindestens 64 Gew.-%, einen Maltitolgehalt von mindestens 6 Gew.-% hat, der Gehalt an Oligo-und Polysacchariden die Differenz auf 100% ausmacht, wobei diese Prozentanteile als Trockenmasse der in der Zusammensetzung vorhandenen Polyole angegeben sind.
